# EUROPEAN PATENT APPLICATION

(11) **EP 0 576 000 A1**
(43) Date of publication of application: **29.12.1993**
(21) Application number: 93110089.5
(22) Date of filing: 24.06.1993
(51) Int. Cl.: A61M 15/00, A61M 16/08

(54) **Device for administering drugs, particularly for aerosol therapy and the like**

(30) Priority: 26.06.1992 IT MI921573
(71) Applicant: DAR SOCIETA' PER AZIONI, I-41037 Mirandola (Modena) (IT)
(72) Inventor: Gibertoni, Lucio, I-41037 Mirandola (IT)
(74) Representative: Modiano, Guido, Dr.-Ing.

(57) **Abstract**

Device for administering drugs particularly for aerosol therapy and the like including a circuit having a branch for inhalation of the air/drug mixture and an expiration branch. The inhalation branch has a first check valve (1) connected to a sanitary-air distribution system and arranged on a corrugated infeed duct (2); a nebulizer (3) is inserted at one end of the infeed duct (2) and introduces at least one nebulized drug into the duct (2); the duct (2) is connected to a mouthpiece (9) through a second check valve (7). The expiration branch comprises a discharge duct (10) connected to the mouthpiece by means of a third check valve (11). The discharge duct (10) has an antibacterial filter (12) which filters expired bacterial loads and discharges the filtered expired air to the outside. The device further comprises expandable compensation means (14) arranged on the corrugated infeed duct (2), connected to the infeed duct (2) by means of at least one opening (15) formed in the infeed duct (2), and compensating the expiration phase of a patient.

## Description

The present invention relates to a device for administering drugs, particularly for aerosol therapy and the like.

As is known, circuits having a branch for the inhalation of the air/drug mixture and an expiration branch are currently used to administer drugs by aerosol or nebulization of liquid compounds in air.

The inhalation branch has a duct for the continuous introduction of air arriving from a circuit for distributing sanitary air, and a drug nebulizer which sprays the nebulized drug into the infeed duct; the mixture is pushed by the air into an administration mouthpiece. In order to avoid contaminations upstream of the duct containing the drug and to prevent that the expired air of the patient contaminates the air/drug mixture, check valves are arranged at the beginning of the duct and at the connection between the duct and the mouthpiece.

In the expiration branch, a discharge duct extends from the administration mouthpiece and is connected to the mouthpiece by another check valve, whereon an antibacterial filter is inserted. The filter is capable of eliminating both the bacterial loads and the fractions of the drug which have not been assimilated from the expired air.

The problem is indeed linked to non assimilated fractions of the drug which are expired by the patient. In fact, the above described circuit requires an amount of drug which is far greater than the indispensable dose, since the inflow of air into the infeed duct is continuous, whereas respiration is discontinuous and has expiration periods which are usually longer than the inspiration periods, causing densification of the nebulized substance in the sanitary air to be inspired, which does not allow for the complete assimilation of the substance.

This problem becomes worse in the case of high-cost drugs used in particular therapies, such as, for example, pentamidine used in the treatment of acquired immunodeficiency syndrome.

The aim of the present invention is to eliminate or substantially reduce the drawbacks described above by providing a device for administering drugs, particularly for aerosol therapy and the like, which reduces the amount of drug to be introduced into the nebulizer to an amount assimilated by the patient.

Within the scope of this aim, an object of the present invention is to provide a device which allows to eliminate the fractions of drug which have not been assimilated by the patient.

Another object is to provide a device which is highly reliable, relatively easy to manufacture and at competitive costs.

This aim, these objects and others which will become apparent hereinafter are achieved by a device for administering drugs particularly for aerosol therapy and the like, according to the invention, comprising a circuit having a branch for the inhalation of the air/drug mixture and an expiration branch, said inspiration branch having a first check valve which is connected to a sanitary-air distribution system and is arranged on a corrugated infeed duct, a nebulizer being inserted at one end of said infeed duct and being suitable to introduce at least one nebulized drug into said duct, said duct being connected to a mouthpiece through a second check valve, said expiration branch comprising a discharge duct which is connected to said mouthpiece by means of a third check valve and has an antibacterial filter which is suitable to filter expired bacterial loads and to discharge the filtered expired air, characterized in that expandable compensation means are arranged on said corrugated duct, are connected to said infeed duct by means of at least one opening formed in said infeed duct, and are suitable to compensate the expiration phase of a patient.

Further characteristics and advantages of the invention will become apparent from the following description of a preferred, but not exclusive, embodiment thereof illustrated only by way of non-limitative example in the accompanying drawings, wherein:
figure 1 is a lateral elevation view of a device according to the invention, at the beginning of the inhalation phase;
figure 2 is a lateral elevation view of a device according to the invention, at the end of the inhalation phase;
figure 3 is a lateral elevation view of a device according to the invention, at the beginning of the expiration phase; and
figure 4 is a lateral elevation view of a device according to the invention, at the end of the expiration phase.

With reference to the accompanying figures, a device for administering drugs particularly for aerosol therapy and the like comprises a circuit having a branch for the inhalation of the air/drug mixture and an expiration branch.

The inhalation branch has a first check valve 1 which allows the infeed of external air after the patient has inspired the air together with the nebulized substance contained in the bag 14, during the inspiratory phase. Valve 1 closes at the end of the inspiratory phase.

A first three-way connector 4 is connected to the other end of a duct 2, and a nebulizer 3 is connected to the connector 4 and introduces at least one nebulized drug into the duct 2. The nebulizer 3 is provided with its own oxygen connection tube 5 which ends with a universal soft connector 6.

A second check valve 7 is connected to the remaining outlet of the connector 4 and is connected, by means of a second three-way connector 8, to an administration mouthpiece 9.

The expiration branch comprises a discharge duct 10 which is connected to the mouthpiece 9 by means of the second connector 8 through a third check valve 11. The discharge duct 10 has, in its body, an antibacterial filter 12 which filters the expired bacterial loads and discharges the filtered expired air to the outside.

The present invention provides, on the corrugated infeed duct 2, expandable compensation means which are constituted, for example, by a bag 14 which is arranged coaxially to the duct 2 and is sealingly welded onto said duct downstream of the first valve 1 and upstream of the first three-way connector 4.

The interior of the bag 14 is connected to the interior of the duct 2 by means of at least one opening formed in said duct, in order to compensate the expiration phase of a patient, since the sanitary air and the oxygen for the nebulization are supplied continuously by the distribution system.

Preferably, multiple slotted openings 15 are formed in the duct 2, which introduce sanitary air into said bag during expiration and draw air from said bag during inhalation. The slotted openings 15 are conveniently arranged so that their main axis is parallel to the axis of the corrugated duct 2.

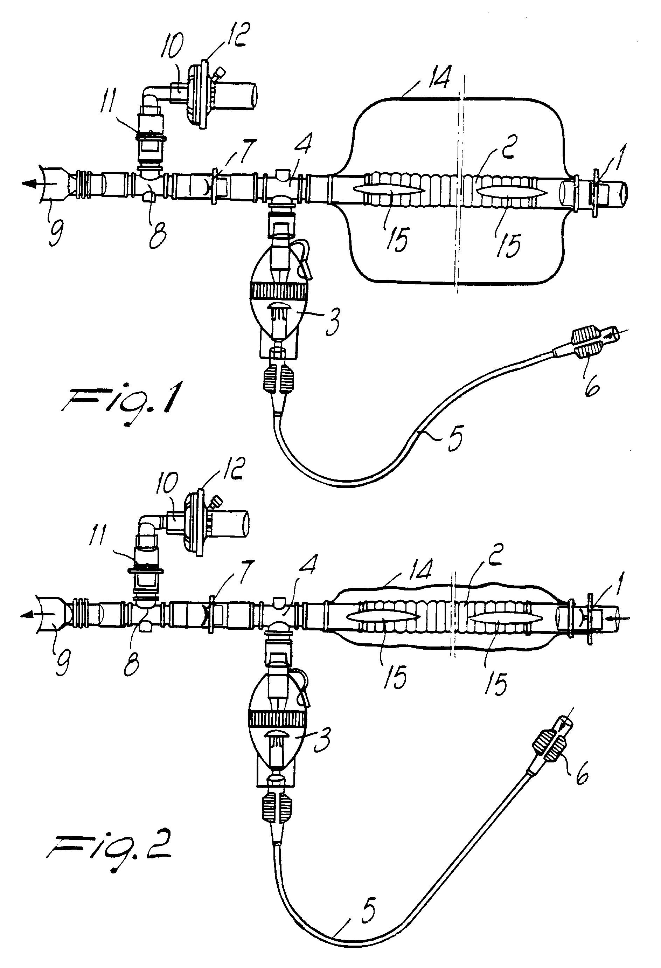

The operation of the device according to the present invention is as follows. At the beginning of the inhalation (figure 1), the circuit has an almost fully inflated bag and the nebulizer is running. The patient, by inhaling the nebulized gas in the bag, creates a negative pressure in the circuit, which closes the third check valve 11 and opens the second check valve 7, whereas the first check valve 1 remains closed until the bag has emptied. Since the volume of the oxygen, mixed with the nebulized substance, is less than the volume required by the patient to complete the inhalation phase, air enters from the outside, since the valve 1 opens (figure 2). This air, by entering, in addition to completing the respiratory cycle, removes all the residues of oxygen and the nebulized substance from the tube 2 of the bag 14.

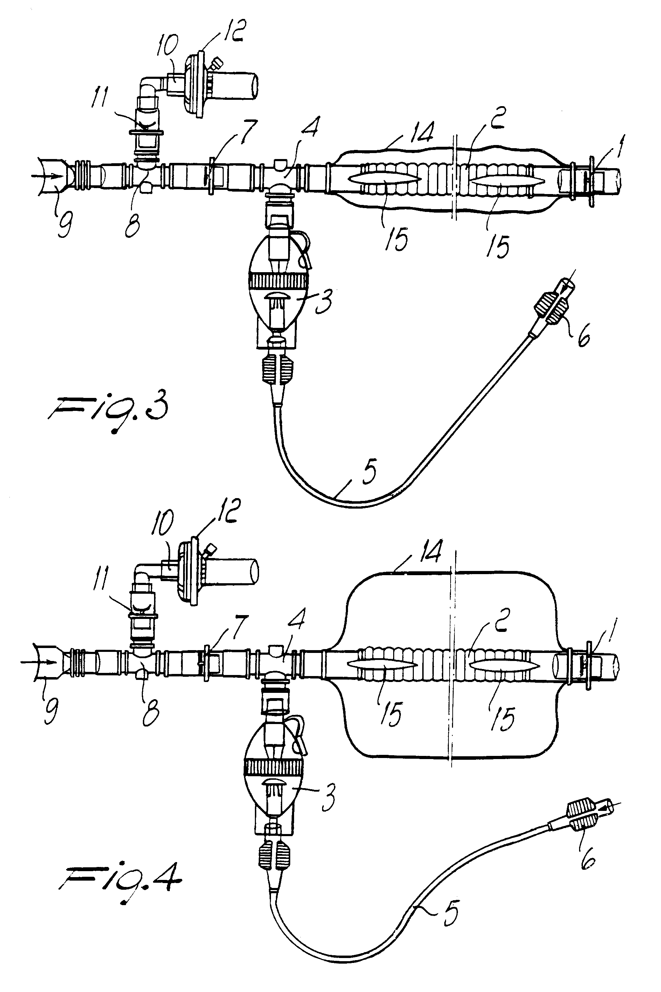

During the expiratory phase, the bag of the circuit is completely empty (figure 3) and the nebulizer is running. The patient, by expiring, pressurizes the circuit up to second check valve 7, which closes, whereas third check valve 11 opens and discharges the expired gas into the filter 12 and afterwards, to the outside.

The nebulized substance enters the duct 2, closing the first check valve 1, and therefore fills the bag, as can be seen in figure 4. At the end of the expiratory phase, the bag is almost full of oxygen or air mixed with the nebulized drug in suspension.

The advantageous compensation of the expiration performed by the bag 14 thus allows for the reduction of the amount of the drug introduced in the nebulizer 3, since it is not necessary to permanently saturate the duct 2 with the nebulized drug. This reduction on one hand allows for savings with high-cost drugs, and on the other hand, while continuing to provide the drug in the dose required by the patient, allows for the practical elimination of drug wastes not absorbed by the patient.

Furthermore, conveniently, the sanitary air contained in the bag 14, which in ordinary operating conditions is at the same pressure as the air introduced by the distribution system into the duct 2, is fed back into the duct 2. This is due both to the inhalation, which creates a lower pressure region in the duct 2, and to the atmospheric pressure, which compresses said bag, at least until the atmospheric pressure and the internal pressure of the bag balance each other out, thus facilitating the inspiration of the patient.

Furthermore, advantageously, the dimensions of the bag may be calibrated to a fraction of the average inspiration or to other values, in order to optimize the compensation between the inhalation phase and the expiration phase.

Practical tests have shown that the device according to the present invention achieves the intended aim and objects, substantially decreasing the wasting of the drug not taken in by the patient and furthermore improving the inhalation and expiration phases of said patient.

The device thus conceived is susceptible to numerous modifications and variations, all of which are within the scope of the inventive concept. All the details may furthermore be replaced with other technically equivalent elements.

In practice, the materials employed, as well as the dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Device for administering drugs particularly for aerosol therapy and the like, comprising a circuit having a branch for inhalation of the air/drug mixture and an expiration branch, said inspiration branch having a first check valve (1) connected to a sanitary-air distribution system and arranged on a corrugated infeed duct (2), a nebulizer (3) inserted at one end of said infeed duct (2) and being adapted to introduce at least one nebulized drug into said duct (2), said duct (2) being connected to a mouthpiece (9) through a second check valve (7), said expiration branch comprising a discharge duct (10) connected to said mouthpiece (9) by means of a third check valve (11) and having an antibacterial filter (12) suitable to filter expired bacterial loads and discharge the filtered expired air, characterized in that it comprises expandable compensation means (14) arranged on said corrugated duct (2), the interior of said expandable compensation means being connected to the interior of said infeed duct (2) by means of at least one opening (15) formed in said infeed duct (2), said expandable compensation means being suitable to compensate the expiration phase of a patient.

2. Device according to claim 1, characterized in that said expandable compensation means comprise a bag (14) which is arranged coaxially to said infeed duct (2) and is sealingly welded onto said duct (2), between said first check valve (1) and said nebulizer (3).

3. Device according to one or more of the preceding claims, characterized in that said at least one opening comprises multiple slotted openings (15) formed in said duct (2) and suitable to introduce sanitary air into said bag (14) during expiration and to draw air from said bag (14) during inhalation.
